(19) Europäisches Patentamt
European Patent Office
Office européen des brevets

(11) EP 3 960 173 B1

(12) # EUROPEAN PATENT SPECIFICATION

(45) Date of publication and mention of the grant of the patent:
**21.08.2024 Bulletin 2024/34**

(21) Application number: **20795446.2**

(22) Date of filing: **24.04.2020**

(51) International Patent Classification (IPC):
***A61K 31/4745*** (2006.01) ***A61K 31/4375*** (2006.01)
***A61K 31/438*** (2006.01) ***A61K 31/44*** (2006.01)
***C07D 471/04*** (2006.01) ***A61P 1/02*** (2006.01)
***A61P 31/12*** (2006.01) ***A61P 31/16*** (2006.01)
***A61P 31/22*** (2006.01) ***A61P 31/20*** (2006.01)
***A61P 31/14*** (2006.01)

(52) Cooperative Patent Classification (CPC):
**A61P 31/14; A61K 31/4745; A61P 31/16; A61P 31/20; A61P 31/22; C07D 471/04**

(86) International application number:
**PCT/CN2020/086835**

(87) International publication number:
**WO 2020/216349 (29.10.2020 Gazette 2020/44)**

(54) **ENTEROVIRUS INHIBITOR**
ENTEROVIRUS-INHIBITOR
INHIBITEUR D'ENTÉROVIRUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.04.2019 CN 201910346258**

(43) Date of publication of application:
**02.03.2022 Bulletin 2022/09**

(73) Proprietor: **Academy of Military Medical Sciences**
**Beijing 100850 (CN)**

(72) Inventors:
- **ZHONG, Wu**
  **Beijing 100850 (CN)**
- **CAO, Ruiyuan**
  **Beijing 100850 (CN)**
- **CHENG, Tong**
  **Xiamen, Fujian 361005 (CN)**
- **XIA, Ningshao**
  **Xiamen, Fujian 361005 (CN)**
- **LI, Song**
  **Beijing 100850 (CN)**

(74) Representative: **Weickmann & Weickmann PartmbB**
**Postfach 860 820**
**81635 München (DE)**

(56) References cited:
**WO-A1-2011/011716**
**WO-A1-2013/154778**
**WO-A1-2018/017426**
**WO-A1-2021/207632**
**US-A1- 2016 339 030**

- **TIANLONG HAO ET AL: "Design, synthesis and pharmacological evaluation of a novel mTOR-targeted anti-EV71 agent", EUROPEAN JOURNAL OF MEDICINAL CHEMISTRY, vol. 175, 24 April 2019 (2019-04-24), AMSTERDAM, NL, pages 172 - 186, XP055746060, ISSN: 0223-5234, DOI: 10.1016/j.ejmech.2019.04.048**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 960 173 B1

## Description

The present application is based on and claims the benefit of priority from Chinese application No. 201910346258.X, filed on April 26, 2019.

## Technical Field

The present application belongs to the field of medical technology, and specifically relates to 9-(6-amino-pyridin-3-yl)-1-(3-trifluoromethyl-phenyl)-1*H*-benzo[*h*][1,6]naphthyridin-one (Formula I), a stereoisomer, a pharmaceutically acceptable salt and/or a solvate and/or a hydrate thereof and a pharmaceutical compositions containing the above compound for use in the treatment of a disease caused by enterovirus infection, as defined in the claims.

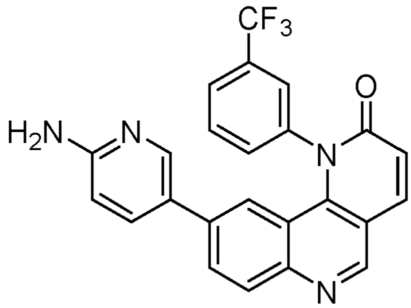

Formula I

## Background Art

Enteroviruses belong to the *Enterovirus* genus of the *Picornaviridae* family, are non-enveloped, positive-sense single-stranded RNA viruses, mainly include polioviruses, group A Coxsackie viruses, and group B Coxsackie viruses, Echo viruses and new enteroviruses, among which Coxsackie viruses and new enteroviruses are the main pathogens of hand-foot-and-mouth disease. Enterovirus infection can cause serious neurological complications like polio, which is the main cause of severe cases and deaths of hand-foot-and-mouth disease. Hand-foot-and-mouth disease is mainly prevalent in the Asia-Pacific region, and many regions/countries including Taiwan, Singapore and Vietnam have large-scale epidemics. In recent years, hand-foot-and-mouth disease has continued to spread in Chinese mainland, severe cases and deaths have been increasing year by year, and it has become a public health problem that seriously threatens the health of children and social stability in China, and it has attracted great attention from all sectors of the community. Generally speaking, vaccines are an important means to combat viral infectious diseases, but hand-foot-and-mouth disease is generally caused by multiple enterovirus infections, and a vaccine can only combat one enterovirus and is lack of cross-protection effect.

In recent years, frequent outbreaks of influenza have severely endangered the lives and health of humans and animals. The highly pathogenic avian influenza that broke out at the end of 2003 not only caused the deaths of tens of millions of poultry and wild birds worldwide, but also caused more than 250 infectious deaths, with a mortality rate as high as 60%. A new type of influenza A, which originated in North America in April 2009, quickly swept the world and caused more than 10,000 deaths. Although humans have been aware of influenza for nearly a century, the existing medical technology for preventing and treating influenza (especially highly pathogenic influenza) is still very weak. There are few drugs that can be used for influenza prevention and treatment. The main ones are three-class drugs, that are M2 ion channel blockers amantadine and rimantadine, neuraminidase inhibitors oseltamivir (commonly known as "Tamiflu") and zanamivir, as well as broad-spectrum antiviral drug ribavirin. Amantadine has been used clinically for many years, and many subtypes of influenza strains have developed severe drug resistance to it. Tamiflu is expensive, and reports of virus resistance to it are increasing.

Human rhinoviruses (HRV) belong to the *Picornaviridae* family, are a group of single-stranded small RNA viruses, and more than 100 serotypes have been found so far. HRV is the main cause of the common cold in humans, and acute and chronic bronchitis and other respiratory diseases are also related to it. Although rhinovirus infection is self-limiting, patients can develop complications such as asthma, congestive heart failure, bronchiectasis, and cystic fibrosis. Especially for children and people with underlying diseases, HRV infection can cause serious sequelae. Due to the large number of HRV serotypes, prevention of HRV infection through vaccination is theoretically unfeasible. It was once thought that interferon (IFN) can be used as the main substance to control HRV and other ribonucleic acid virus infections, but IFN treatment is not effective for the established HRV infection.

The herpesvirus family is a kind of double-stranded DNA virus with an envelope structure. Herpes simplex virus (HSV) belongs to the $\alpha$ subfamily, which exists widely in nature and can infect humans and many animals, and especially has a strong tropism for human skin tissues. Herpesvirus mainly infects the host through skin, mucous membranes and nerve tissues and causes corresponding pathological changes, and is a common pathogen of human viral diseases. Herpes simplex virus can be divided into two serotypes, HSV-1 and HSV-2. HSV-1 infects the human body mainly to cause herpes labialis, pharyngitis, keratitis, and can also cause sporadic encephalitis and other serious diseases. HSV-2 mainly causes genital herpes through the infection of damaged skin and mucous membrane. In recent years, it has been found in epidemiological investigations that HSV-1 and HSV-2 are equally important among the pathogens that cause genital herpes, and both can be latent in the body for a long time. During the latent infection process, the structure and function of the herpes virus genome are not affected or destroyed in any way, and a series of regulations related to viral gene transcription and expression are in a state of stagnation. In this process, there is no complete genome replication, but the limited local gene transcription exists, and enters the stage of proliferative replication infection under certain conditions. At present, the incidence of genital herpes is rapidly increasing and it is easy to recur, which has caused great difficulties in the treatment and prevention of related diseases.

Vesicular stomatitis viruses (VSV) can cause vesicular stomatitis, is a kind of pathogen of highly contagious anthropozoonosis. The clinical symptoms after its onset are very similar to that of foot-and-mouth disease, swine vesicular disease and swine vesicular herpes, and it is usually difficult to distinguish them. The main clinical features are the appearance of small vesicles, ulcers and scabs on the lips, coronets and breasts. The pain caused by erosions and ulcers results in the loss of appetite and secondary mastitis in animals, which leads to reduced productivity and even death of animals, causing serious economic losses to the breeding industry. Meanwhile, VSV can also infect humans, causing flu-like symptoms and even deaths in severe cases.

In summary, the development of a specific small molecule as broad-spectrum antiviral drug is urgent. Torin compound is an ATP analogue, which is a serine/threonine protein kinase, also known as protein kinase B. It is an important factor in the mTOR/AKT signal pathway, plays an important role in a series of physiological activities such as cell proliferation, differentiation, apoptosis and metabolism, and is closely related to the occurrence of tumors. At present, the compound has undergone preclinical experiments.

The hand-foot-and-mouth disease (HFMD) mentioned above is a childhood infectious disease caused by a variety of human enteroviruses, it spread through fecal-oral or respiratory droplets, and the infection may also occur through contact with an infected person's skin or fluid from blisters. The viruses that cause hand-foot-and-mouth disease belongs to the human enterovirus (HEV), which can be divided into four subtypes: HEV-A, HEV-B, HEV-C and HEV-D, according to serotype classification, in which Enterovirus71 (EV71) and Coxsackie virus group A type 16 (Cox Asckievirus 16, CA16) are the most common. In the past half century, there have been many outbreaks of hand-foot-and-mouth disease in many places around the world, spreading across all continents, making it a worldwide disease of concern. In the 1990s, Asia encountered a peak of outbreak of hand-foot-and-mouth disease, especially in Southeast Asia, where a large-scale epidemic of hand-foot-and-mouth disease, dominated by EV71 infection, caused central nervous system symptoms and led to a large increase in deaths.

At present, there is no specific antiviral drug on the market that can effectively inhibit enteroviruses. The anti-enteroviral drugs currently in preclinical research mainly include the following categories: 1) antiviral drugs that target the adsorption and entry phases of viruses, such as soluble human scavenger receptor class B member 2 (SCARB2), P-selectin glycoprotein ligand-1 (PSGL-1), heparin or heparin analogs, and saliva acid; 2) antiviral drugs that target the uncoating phase of viruses, such as uncoating inhibitors WIN51711, BPROZ-194, and BPROZ-112; 3) antiviral drugs that target the RNA translation phase of viruses, such as quinacrine, and amantadine; 4) antiviral drugs that target the protein processing phase of viruses, such as substrate analogue of 2A protease LVLQTM and irreversible peptidase inhibitor of 3C protein AG7088; however, most of them act on a single viral subtype and do not have broad-spectrum characteristics, and due to the high variability of enteroviruses, antiviral drugs that target the virus itself are prone to cause drug resistance. In addition, some vaccines against enteroviruses are also under development, such as recombinant VP1 protein vaccines and recombinant DNA vaccines. However, due to the short application time and low application scale, there is still a lack of sufficient clinical feedback.

Tianlong Hao et al. disclose in Eur. J. Med. Chem., 2019, 172-186, that hand-foot-and-mouth disease (HFMD) is an infectious disease caused by enteroviruses; Enterovirus 71 (EV71) is the main pathogen that causes HFMD. The mTOR inhibitor Torin2 has significant and-EV71 activity and suitable bio-availability.

**Contents of the Application**

The purpose of the present application is to provide a new type of enterovirus inhibitor in view of the scarcity of drugs for clinical treatment of enteroviruses.

In the present application, it has been discovered through creative researches that 9-(6-amino-pyridin-3-yl)-1-(3-trifluoromethyl-phenyl)-1H-benzo[h][1,6]naphthyridin-one,

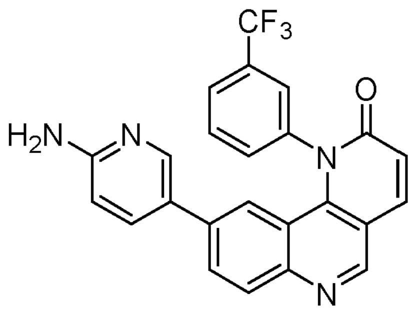

Formula I

has the function of protecting the cell infected by enterovirus, adenovirus, influenza virus, rhinovirus, herpes virus, vesicular stomatitis virus and herpes zoster virus, and of inhibiting virus replication, and shows very good effects in the treatment of the disease caused by enterovirus, adenovirus, influenza virus, rhinovirus, herpes virus, vesicular stomatitis virus and herpes zoster virus; in particular, it has significant anti-enteroviral activity and can be developed as a new anti-enteroviral drug, and thus has a wide range of application prospects.

The compound 9-(6-amino-pyridin-3-yl)-1-(3 -trifluoromethyl-phenyl)-1H-benzo[h][1,6]naphthyridin-one can significantly inhibit the proliferation of enteroviruses EV-D68, Poliovirus I/II/III, CB3 and EV71 viruses in host cells.

The compound 9-(6-amino-pyridin-3-yl)-1-(3-trifluoromethyl-phenyl)-1H-benzo[h][1,6]naphthyridin-one can target the mTOR protein of host cells, and then regulate the PI3K/Akt/mTOR signal pathway, thereby inhibiting the replication of enteroviruses, has obvious antiviral activity, and can be used to prepare an anti-enteroviral drug.

The first aspect of the present application provides a compound represented by Formula I, or a pharmaceutically acceptable salt, a stereoisomer, a hydrate or a solvate thereof, for use in the treatment of an infectious disease caused by a virus, wherein the virus is an enterovirus, and wherein the enterovirus is an EV-D68, a Poliovirus I/II/III or a CB3 virus.

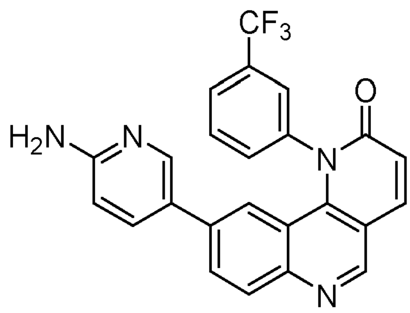

Formula I.

The second aspect of the present application provides a pharmaceutical composition, which comprises the compound of Formula I described in the present application, a pharmaceutically acceptable salt, a stereoisomer, a hydrate, or a solvate thereof, for use in the treatment of an infectious disease caused by a virus, wherein the virus is an enterovirus, and wherein the enterovirus is an EV-D68, a Poliovirus I/II/III or a CB3 virus.

In a preferred embodiment, the pharmaceutical composition described in the second aspect of the present application further comprises a pharmaceutically acceptable carrier or excipient. The pharmaceutical composition can be made into a solid preparation, an injection, an external preparation, a spray, a liquid preparation or a compound preparation as required.

According to the present application, the compound of Formula I can protect a cell from the cytopathic effect (CPE) caused by virus infection, inhibit virus replication in the cell, reduce the viral nucleic acid load in cell culture, and can also provide complete protection for a virus-infected mouse.

After long-term research, the inventors of the present application discovered some new features of the compound of Formula I in cells:

First, in the in vitro antiviral experiment, the compound of Formula I can reduce the CPE level of the cells infected by enterovirus, adenovirus, influenza virus, rhinovirus, herpes virus, vesicular stomatitis virus and herpes zoster virus at micromolar concentration;

Second, the compound of Formula I can reduce the viral nucleic acid load level of the cells infected by enterovirus,

adenovirus, influenza virus, rhinovirus, herpes virus, vesicular stomatitis virus and herpes zoster virus at micromolar concentration;

Third, the compound of Formula I can provide complete protection to the mice infected by enterovirus, adenovirus, influenza virus, rhinovirus, herpes virus, vesicular stomatitis virus and herpes zoster virus at micromolar concentration.

Also disclosed (but not part of the claimed invention) is the compound of Formula I, a pharmaceutically acceptable salt, a stereoisomer, a hydrate or a solvate thereof, or the pharmaceutical composition described in the present application, for use in the treatment of an infectious disease caused by a virus such as enterovirus (other than those claimed), polio virus (other then those claimed), adenovirus, influenza virus, rhinovirus, herpes simplex virus, vesicular stomatitis virus, and/or herpes zoster virus.

Also disclosed (but not part of the claimed invention) is the compound of Formula I, a pharmaceutically acceptable salt, a stereoisomer, a hydrate or a solvate thereof, or the pharmaceutical composition described in the present application, for use in inhibiting the replication of enterovirus, adenovirus, influenza virus, rhinovirus, herpes simplex virus, vesicular stomatitis virus and/or herpes zoster virus in a host cell (e.g., a cell of mammal).

Also disclosed (but not part of the claimed invention) is the use of the compound of Formula I, a stereoisomer, a hydrate or a solvate thereof described in the present application for inhibiting the replication of an enterovirus in a target cell.

In some aspects, the enterovirus described in the present application is a virus of HEV-A, HEV-B, HEV-C, or HEV-D subtype.

In some aspects, the enterovirus described in the present application is an EV-D68, a Poliovirus I/II/III, a CA16, a CA6, a CA10, a CB3, or an EV71 virus.

In some aspects, the enterovirus described in the present application is an EV-D68, a Poliovirus I/II/III, a CB3 or an EV71 virus.

According to the invention, the enterovirus is an EV-D68, a Poliovirus I/II/III or a CB3 virus.

In some embodiments, the enterovirus described in the present application is an EV-D68 or a CB3 virus.

In some embodiments, the enterovirus described in the present application is an EV-D68.

In some embodiments, the enterovirus described in the present application is a Poliovirus I/II/III.

In some non-claimed aspects, the enterovirus described in the present application is a CA16 virus.

In some non-claimed aspects, the enterovirus described in the present application is a CA6 virus.

In some non-claimed aspects, the enterovirus described in the present application is a CA10 virus.

In some embodiments, the enterovirus described in the present application is a CB3 virus.

In some non-claimed aspects, the enterovirus described in the present application is an EV71 virus.

In the present application, Poliovirus I/II/III refers to any type or a mixed strain of any two types or a mixed strain of any three types selected from the group consisting of Poliovirus I, Poliovirus II and Poliovirus III.

The present application also relates to a pharmaceutical composition, comprising a compound of Formula I, a pharmaceutically acceptable salt and/or a pharmaceutically acceptable solvate or a hydrate thereof, and a pharmaceutically acceptable carrier, for use in the treatment of an infectious disease caused by a virus, wherein the virus is an enterovirus, and wherein the enterovirus is an EV-D68, a Poliovirus I/II/III or a CB3 virus.

The pharmaceutically acceptable salt of the compound of Formula (I) described in the present application comprises salts formed with inorganic or organic acids, or salts formed with inorganic or organic bases. The present application relates to all forms of the above-mentioned salts, including: sodium salt, potassium salt, calcium salt, lithium salt, meglumine salt, hydrochloride, hydrobromide, hydriodate, nitrate, sulfate, hydrogen sulfate, phosphate, hydrogen phosphate, acetate, propionate, butyrate, oxalate, trimethylacetate, adipate, alginate, lactate, citrate, tartrate, succinate, maleate, fumarate, picrate, aspartate, gluconate, benzoate, methanesulfonate, ethanesulfonate, benzenesulfonate, p-toluenesulfonate and pamoate.

The pharmaceutical composition described in the present application can be administered through various routes, such as oral tablet, capsule, powder, oral liquid, injection and transdermal preparation. According to conventional pharmaceutical practices, the pharmaceutically acceptable carrier comprises diluent, filler, disintegrant, wetting agent, lubricant, coloring agent, flavoring agent or other conventional additives. Typical pharmaceutically acceptable carriers include, for example, microcrystalline cellulose, starch, crospovidone, povidone, polyvinylpyrrolidone, maltitol, citric acid, sodium laurylsulfonate or magnesium stearate, etc.

The present application relates to a pharmaceutical composition, which comprises the compound of Formula I described in the present application, a pharmaceutically acceptable salt, a stereoisomer, a hydrate, or a solvate thereof, and at least one pharmaceutically acceptable carrier for use in the treatment of an infectious disease caused by an enterovirus, as defined in the claims.

The pharmaceutical composition can be prepared into various forms according to different administration routes. The compound 9-(6-amino-pyridin-3-yl)-1-(3-trifluoromethylphenyl)-1H-benzo[h][1,6]naphthyridin-one can be made into

a variety of pharmaceutically acceptable preparation forms, such as tablet, granule, powder, capsule, oral liquid, injection, for the treatment of anti-enterovirus.

In some embodiments, the mammal comprises bovidae, equidae, caprinae, suidae, canidae, felidae, glires, primate, among which the mammal is preferred to be human.

According to the present application, the pharmaceutical composition can be administered in any one of the following routes: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or administration with the help of an explant reservoir, wherein the preferred administration route is oral, intraperitoneal or intravenous administration.

When orally administered, the compound of Formula I can be prepared into any form of orally acceptable preparation, including but not limited to a tablet, a capsule, an aqueous solution or an aqueous suspension. The carrier for use in a tablet generally includes lactose and corn starch, and a lubricant such as magnesium stearate can also be added. The diluent for use in a capsule generally includes lactose and dry corn starch. The aqueous suspension is usually used by mixing an active ingredient with a suitable emulsifier and a suitable suspending agent. If necessary, a sweetener, a flavoring agent or a coloring agent can also be added to the above-mentioned oral preparation forms.

When rectally administered, the compound of Formula I can generally be prepared in a form of suppository, which is prepared by mixing the drug with a suitable non-irritating excipient. The excipient is present in solid state at room temperature, but melts at the rectal temperature to release the drug. Such excipient includes cocoa butter, beeswax and polyethylene glycol.

When topically administered, especially for treatment of easily accessible diseased parts or organs such as eye, skin, or lower intestinal neurological disease by topical application, the compound of Formula I can be prepared in various forms of topical preparations according to different diseased parts or organs, the specific instructions are as follows:

When topically administered to eye, the compound of Formula I can be formulated into a preparation form such as micronized suspension or solution, the carrier used is isotonic sterile saline with a certain pH, and a preservative such as benzyl chloride alkoxide may or may not be added. In addition, for administration to eye, the compound can also be prepared in a form of ointment such as vaseline ointment.

When topically administered to skin, the compound of Formula I can be prepared into a suitable form such as an ointment, a lotion or a cream, in which the active ingredient is suspended or dissolved in one or more carriers. The carrier for use in an ointment includes, but is not limited to: mineral oil, liquid petrolatum, white petrolatum, propylene glycol, polyethylene oxide, polypropylene oxide, emulsifying wax, and water. The carrier for use in a lotion or a cream includes, but is not limited to: mineral oil, sorbitan monostearate, Tween-60, cetyl ester wax, hexadecenyl aryl alcohol, 2-octyldodecanol, benzyl alcohol and water.

When topically administered to lower intestinal tract, the compound of Formula I can be prepared into a form such as rectal suppository as described above or a suitable enema preparation form, in addition, a topical transdermal patch can also be used.

The compound of Formula I can also be administered in a preparation form of sterile injection, including sterile injectable aqueous solution or oil suspension, or sterile injectable solutions, wherein the usable carrier and solvent includes water, Ringer's solution and isotonic sodium chloride solution. In addition, a sterilized non-volatile oil such as monoglyceride or diglyceride can also be used as solvent or suspension media.

As described herein, "therapeutically effective amount" or "prophylactically effective amount" refers to an amount that is sufficient to treat or prevent a patient's disease but is sufficiently low to avoid serious side effects (at a reasonable benefit/risk ratio) within a reasonable medical judgment. The therapeutically effective amount of the compound will change upon the factors such as the selected specific compound (for example, considering the potency, effectiveness and half-life of compound), the selected route of administration, the disease to be treated, the severity of the disease to be treated, and the conditions such as age, size, weight and physical disease of the patient to be treated, the medical history of the patient to be treated, the duration of treatment, the nature of concurrent therapy, the desired therapeutic effect and so on, but it can still be routinely determined by those skilled in the art.

In addition, it should be noted that the specific dosage and usage of the compound of Formula I described in the present application for different patients depends on many factors, including the patient's age, weight, gender, natural health status, nutritional status, the active strength of the compound, the time of administration, the metabolic rate, the severity of disease, and the subjective judgment of physician. Herein it is preferable to use a dose of 0.01 to 100 mg/kg body weight/day.

The above various preparation forms of drugs can be prepared according to conventional methods in the pharmaceutical field.

In summary, compared with the prior art, the present application has the following advantages and effects:

Compound 9-(6-amino-pyridin-3-yl)-1-(3-trifluoromethyl-phenyl)-1H-benzo[h][1,6]naphthyridin-one can inhibit the infection of cells caused by enterovirus EV-D68, PoliovirusI/II/III, CA16, CA6, CA10, CB3 and EV71 viruses, especially the infection of cells caused by EV-D68, PoliovirusI/II/III, CB3 and EV71 viruses, and has a broad-spectrum antiviral activity. According to the invention, the compound of formula I is for use in the treatment of an infectious disease caused by an enterovirus which is an EV-D68, a Poliovirus I/II/III or a CB3 virus.

Compound 9-(6-amino-pyridin-3-yl)-1-(3-trifluoromethyl-phenyl)-1H-benzo[h][1,6]naphthyridin-one acts on the ATP binding region of mTOR protein in the host cell, can competitively inhibit the binding of ATP to the mTOR protein binding site, block the PI3K/Akt/mTOR signal pathway, and prevent viral transcription and replication.

**Brief Description of the Drawings**

Figure 1 shows a fitting curve of compound concentration-mTOR kinase inhibition rate;

Figure 2 shows the Western blot results of rapamycin and the compound of Formula I.

**Specific Models for Carrying Out the Application**

In order to better understand the content of the present application, the following experiments and results are combined to further illustrate the anti-enteroviral application of compound 9-(6-amino-pyridin-3-yl)-1-(3-trifluoromethyl-phenyl)-1H-benzo[h][1,6]naphthyridin-one.

Example 1

In vitro experiment of anti-enteroviral activity and cytotoxicity of compound 9-(6-amino-pyridin-3-yl)-1-(3-trifluoromethyl-phenyl)-1H-benzo[h][1,6]naphthyridin-one

The in vitro antiviral experiment of the present application involved a variety of subtypes of enterovirus, including EV-D68, Poliovirus I/II/III (mixed vaccine strains of three subtypes), CA16, CA6, CA10, CB3 (all the above strains were provided by the Academy of Military Medical Sciences) and EV71 virus (purchased from ATCC). The specific methods were as follows.

Among them, the activity of EV-D68, Poliovirus I/II/III, CA16, CA6, CA10 and EV71 viruses was tested with RD cells (purchased from ATCC). The experimental method was as follows:

(1) Dissolution of compound

① According to the mass and molecular weight of 9-(6-amino-pyridin-3-yl)-1-(3-trifluoromethyl-phenyl)-1H-benzo[h][1,6]naphthyridin-one, the compound to be tested was dissolved to 100 mM in DMSO.

(2) Screening of antiviral activity

① Cell maintenance solution (DMEM+2%FBS, Gibco, catalog numbers: 11995-065, 1600-044) was used to dilute the compound to be tested to a concentration of 800μM, and then the compound to be tested was subjected to 3-fold gradient dilution to reach a total of 10 concentrations, and then the diluted compound was added to a 96-well plate with white wall and transparent bottom, 50 μl per well. To both the cell control group and the virus control group, an equal volume of the cell maintenance solution was added.

② The virus seeds involved were taken out from -80°C and equilibrated to room temperature.

③ Virus growth medium (DMEM+2% FBS, Gibco, catalog numbers: 11995-065, 1600-044) was used to dilute the virus seeds to 100 TCID50, and the diluted virus seed was added to the above 96-well plate, 50μl per well. An equal volume of virus growth solution was added to the cell control group.

④ The RD cells were inoculated into the above 96-well plate at a concentration of $1*10^5$/ml, 100μl per well, and a final volume of 200μl per well was reached. The final concentration of the drug was 0.25 times the pretreatment concentration.

⑤ The RD cells were cultured at 37°C for 4 days for testing.

⑥ The buffer of CellTiter-Glo® luminescent cell viability assay reagent (Promega) was mixed with the substrate in the dark to prepare a working solution.

⑦ The culture medium in the 96-well plate was discarded, the plate was dried by patting, 100 μl of detection reagent was added to each well, and the 96-well plate was shaken for 4 minutes with orbital shaker to induce cell lysis. After the signal was stabilized for 15 minutes in the dark, the chemiluminescence unit was measured using an MD5 microplate reader (Molecular Devices), and the plate reading program was the CellTiter-Glo preset program.

The formula for calculating the inhibition rate of drug on the virus was: inhibition rate (%) = (value of experimental group - average value of virus group)/(average value of cell control group - average value of virus group)*100
The inhibition rate-concentration curve was fitted to S-curve by using origin8.0 software, and the $IC_{50}$ value of the compound to be tested was calculated.

(3) Determination of cytotoxicity

① The cell maintenance solution was used to dilute the compound to be tested to a concentration of 400 μM, and then the compound to be tested was subjected to 3-fold gradient dilution to reach a total of 10 concentrations.

② The diluted compound was added to a 96-well plate with white wall and transparent bottom, 100μl per well. An equal volume of cell maintenance solution was added to the cell control group.

③ The RD cells was inoculated into the above 96-well plate at a concentration of $1*10^5$/ml, 100μl per well, and a final volume of 200μl per well was reached. The final concentration of drug was 0.5 times the pretreatment concentration.

④ The RD cells were cultured at 37°C for 4 days for testing.

⑤ The Buffer of CellTiter-Glo® luminescent cell viability assay reagent was mixed with the substrate in the dark to prepare a working solution.

⑥ The culture medium in the 96-well plate was discarded, the plate was dried by patting, 100 μl of the detection reagent was added to each well, and the 96-well plate was shaken with an orbital shaker for 4 minutes to induce cell lysis. After the signal was stabilized in the dark for 15 minutes, the chemiluminescence unit was measured, and the plate reading program was the CellTiter-Glo preset program.

The inhibition rate of the drug at each dilution degree was calculated according to the following formula: inhibition rate (%) = (average value of cell control group - value of experimental group)/(average value of cell control group - minimum value of experimental group) * 100.

(4) Data analysis

The inhibition rate-concentration curve was fitted to S-curve by using origin8.0 software, and the $IC_{50}$ value of the compound to be tested was calculated. By using the same method, the $CC_{50}$ value was calculated, and the selection index $SI=CC_{50}/IC_{50}$ was calculated based on $IC_{50}$ and $CC_{50}$.
The activity of CB3 virus was tested with vero cells. The experimental method was as follows:

(1) Dissolution of compound

① According to the mass and molecular weight of 9-(6-amino-pyridin-3-yl)-1-(3-trifluoromethyl-phenyl)-1H-benzo[h][1,6]naphthyridin-one, the compound to be tested was dissolved with DMSO to 100 mM.

(2) Screening of antiviral activity

① Vero cells (purchased from ATCC) were inoculated into a 96-well plate with white wall and transparent bottom at a concentration of $1*10^5$/ml 24 hours in advance, 100μl per well.

② The plate was washed three times with PBS, 200μl per well, and the cell maintenance solution was added at the last time, 100μl/well.

③ The cell maintenance solution (DMEM+2%FBS, Gibco, catalog numbers: 11995-065, 1600-044) was used to dilute the compound to be tested to a concentration of 800μM, and then the compound to be tested was subjected to 3-fold gradient dilution to reach a total of 10 concentrations. Then the diluted compound was added to the above 96-well plate, 50 μl per well. To both the cell control group and the virus control group, an equal volume of the cell maintenance solution was added.

④ The CB3 virus seed was taken out from -80°C and equilibrated to room temperature.

⑤ Virus growth medium (DMEM+2% FBS, Gibco, catalog numbers: 11995-065, 1600-044) was used to dilute the virus seed to 100 TCID50, and the diluted virus seed was added to the above 96-well plate, 50μl per well. An equal volume of the virus growth solution was added to the cell control group, the final volume per well was 200 μl, and the final concentration of drug was 0.25 times the pretreatment concentration.

⑥ The Vero cells were cultured at 37°C for 5 days for testing.

⑦ The Buffer of the CellTiter-Glo® luminescent cell viability assay reagent was mixed with the substrate in the dark to prepare a working solution. The culture medium in the 96-well plate was discarded, the plate was dried by gently patting, 100 μl of detection reagent was added to each well, and the 96-well plate was shaken for 4 minutes with an orbital shaker to induce cell lysis. After the signal was stabilized in the dark for 15 minutes, the chemiluminescence unit was measured, and the plate reading program was the CellTiter-Glo preset program.

The formula for calculating the inhibition rate of drug on the virus was: inhibition rate (%) = (value of experimental group - average value of virus group)/(average value of cell control group - average value of virus group)*100
The inhibition rate-concentration curve was fitted to S-curve by using origin8.0 software, and the $IC_{50}$ value of the compound to be tested was calculated.
(3) Determination of cytotoxicity

① Vero cells were inoculated in a 96-well plate with white wall and transparent bottom at a concentration of $1*10^5$/ml 24 hours in advance, 100μl per well.

② The plate was washed three times with PBS, 200μl per well, and 100μl/well of the cell maintenance solution was added at the last time.

③ The cell maintenance solution was used to dilute the compound to be tested to a concentration of 400μM, and then the compound to be tested was subjected to 3-fold gradient dilution to reach a total of 10 concentrations. The diluted compound was added to the above 96-well plate, 100μl per well, the final volume per well was 200μl, and the final concentration of drug was 0.5 times the pretreatment concentration. An equal volume of the cell maintenance solution was added to the cell control group.

④ The Vero cells were cultured at 37°C for 5 days for testing.

⑤ The Buffer of CellTiter-Glo® luminescent cell viability assay reagent was mixed with the substrate in the dark to prepare a working solution.

⑥ The culture medium in the 96-well plate was discarded, the plate was dried by patting, 100 μl of the detection reagent was added to each well, and the 96-well plate was shaken for 4 minutes with an orbital shaker to induce cell lysis. After the signal was stabilized for 15 minutes in the dark, the chemiluminescence unit was measured using an MD5 microplate reader (Molecular Devices), and the plate reading program was the CellTiter-Glo preset program.

The inhibition rate of the drug at each dilution degree was calculated according to the following formula: inhibition rate (%) = (average value of cell control group - value of experimental group)/(average value of cell control group - minimum value of experimental group)*100

(4) Data analysis

The inhibition rate-concentration curve was fitted to S-curve by using origin8.0 software, and the $IC_{50}$ value of the compound to be tested was calculated. The $CC_{50}$ value was calculated by using the same method, and the selection index $SI=CC_{50}/IC_{50}$ was calculated based on $IC_{50}$ and $CC_{50}$.

The results of the inhibitory activity of compound 9-(6-amino-pyridin-3-yl)-1-(3-trifluoromethyl-phenyl)-1H-benzo[h][1,6]naphthyridin-one against the virus were shown in Table 1 below:

Table 1: Inhibitory activity and cytotoxicity of compound against enterovirus

| Subtype of enterovirus | $IC_{50}$(μM) | $CC_{50}$(μM) | SI($CC_{50}$/ $IC_{50}$) |
|---|---|---|---|
| EV71 | 0.01±0.00 | 0.04±0.01 | 4 |
| EVD68 | 0.005±0.00 | 0.37±0.01 | 74 |
| CA16 | > 10.0±0.00 | 0.37±0.01 | - |
| CB3 | 0.005±0.00 | 0.37±0.01 | 74 |
| CA6 | > 10.0±0.00 | 0.37±0.01 | - |
| CA10 | > 10.0±0.00 | 0.37±0.01 | - |
| Polio I/II/III | 0.052±0.00 | 0.37±0.01 | 7.1 |

The experimental results showed that the compound 9-(6-amino-pyridin-3-yl)-1-(3-trifluoromethyl-phenyl)-1H-benzo[h][1,6]naphthyridin-one had a significant inhibitory effect on enteroviruses EV-D68, Poliovirus I/II/III, CB3 and EV71, and had a high selectivity safety index (Table 1). This showed that the compound had strong inhibitory activity against the two enteroviruses EVD68 and CB3, and had a certain selectivity at the same time. Only the the treatment of an infectious disease caused by an EV-D68, a Poliovirus I/II/III or a CB3 virus is according to the invention.

Example 2

Experiment of inhibitory activity of 9-(6-amino-pyridin-3-yl)-1-(3-trifluoromethyl-phenyl)-1H-benzo[h][1,6]naphthyridin-one on mTOR kinase

Experimental method:

Preparation of reaction buffers:

Basic buffer composition: 50mM (mmol/L) HEPES (pH 7.5), 1mM EGTA, 0.01% Tween-20, 10 mM $MnCl_2$, 2mM DTT (diluted from 500 mM when used).

① Substrate buffer solution: 1650 μL of 2.5× substrate buffer solution was composed of 1559.6 μL of 1× basic buffer, 89.2 μL of GFP-4E-BP1 (18.5 μM stock solution, purchased from Thermo Fisher, catalog number: PV4759) and 1.2 μL of ATP (10 mM), and the final concentrations were 0.4 μM GFP-4E-BP1 and 3 μM ATP.

② mTOR kinase buffer solution: 1650 μL of 2.5× mTOR kinase buffer solution was composed of 1640.2 μL of 1× basic buffer, and 9.8 μL of mTOR (0.21 mg/mL stock solution), and the final concentration was 0.5 μg/mL.

③ Detection buffer solution: 3960 μL of 2× detection buffer solution was composed of 3797.1 μL of TR-FRET buffer diluent (purchased from Thermo Fisher, catalog number: PV3574), 4.5 μL of Tb-anti-p4E-BP1 antibody (3.49 μM stock solution, purchased from Thermo Fisher, catalog number: PV4757), and 158 μL of EDTA (500 mM stock solution), and the final concentrations were 2 nM Tb-anti-p4E-BP1 antibody and 10 mM EDTA.

Experimental steps:

① 20 μL of 100% DMSO solution containing 5 mM compound to be tested was added to a 96-well plate.

② The compound was serially diluted with DMSO for 3 times.

③ 1 μL of the compound in the previous step was taken, diluted with 19 μL of mTOR kinase buffer, and transferred to another 96-well plate.

④ 4 μL of mTOR kinase solution (purchased from Thermo Fisher, catalog number: PV4753) was added to the 384-well plate.

③ 2 μL of the compound obtained in ③ was taken out and added to a 384-well plate with mTOR kinase solution, and incubated at room temperature for 15 minutes.

⑥ 4 μL of the substrate solution was added to initiate the reaction.
The final concentrations of mTOR reaction solutions were: 0.5 μg/mL mTOR, 0.4 μM GFP-4E-BP1, 3 μM ATP. The final concentrations of the compound to be tested were: 50000, 16666, 5555, 1851, 617.3, 205.8, 68.58, 22.86, 7.62, 2.54 and 0.85 nM. The final concentration of the DMSO solution was 1%.

⑦ Incubation was performed for 60 minutes at room temperature.

⑧ 10 μL of the detection buffer was added. The final concentrations were 2 nM Tb-anti-p4E-BP1 antibody and 10 mM EDTA.

⑨ Incubation was performed for 30 minutes at room temperature.

⑩ TR-FRET value was read on an MD5 multi-mode plate reader (Molecular Devices). The excitation wavelength was 340 nm, the emission wavelength 1 was 495 nm, and the emission wavelength 2 was 520 nm. The ratio of 520nm/495nm readings was calculated as the TR-FRET value.

Data processing:

The $IC_{50}$ of compound was fitted by nonlinear regression equation:

$$Y = Bottom+(Top-Bottom)/(1+10^{\wedge}((LogIC_{50}-X)*HillSlope));$$

X: common logarithm value of compound concentration; Y: TR-FRET value (520nm/495nm).
The experimental results were shown in Table 2 and Figure 1.

Table 2: Inhibitory activity of compounds on mTOR kinase

| | Bottom | Top | HillSlope | mTOR $IC_{50}$ (nM) |
|---|---|---|---|---|
| Compound | 3.74 | 103.60 | 1.14 | 3.01 |

The experimental results of Table 2 and Figure 1 showed that the compound 9-(6-amino-pyridin-3-yl)-1-(3-trifluoromethyl-phenyl)-1H-benzo[h][1,6]naphthyridin-one had a strong inhibitory activity on mTOR kinase, with an $IC_{50}$ of 3.01 nM, which further verified its mechanism that viral activity was inhibited by inhibiting mTOR kinase activity.

Example 3

Experiment of inhibitory activity of compound 9-(6-amino-pyridin-3-yl)-1-(3-trifluoromethyl-phenyl)-1H-benzo[h][1,6] naphthyridin-one on mTORC1 and mTORC2

In order to test the inhibitory activity of the synthesized compound 9-(6-amino-pyridin-3-yl)-1-(3-trifluoromethyl-phenyl)-1H-benzo[h][1,6]naphthyridin-one on the two mTOR complexes, the inhibitory activity experiment of mTORC1 and mTORC2 was performed. Since mTORC1 and mTORC2 exerted function by activating the phosphorylation of downstream substrates, the inhibitory activity of the compound on mTORC1 and mTORC2 could be determined by detecting the phosphorylation levels of Thr389 site of mTORC1 downstream substrate p70S6K1 and Ser473 site of mTORC2 downstream substrate Akt.

Experimental method:

Pretreatment of compound:

① The RD cells were cultured in DMEM medium containing 10% FBS and 1×PS (penicillin and streptomycin at 100 IU and 100 μg/mL, respectively) at 37°C and 5% $CO_2$.

② The RD cells ($5\times10^5$ cells/2 mL medium) were inoculated into a 6-well plate and incubated at 37°C and 5% $CO_2$ for 24 hours.

③ The cells were washed once with PBS, and the cells were cultured in serum-free medium without nutrients overnight.

④ Formulation of compound:

Formulation of insulin medium: insulin was diluted in DMEM medium containing 10% FBS and 1×PS so that the final concentration of insulin was 167 nM.

Pretreatment of compound to be tested: the compound was dissolved in DMSO so that the concentration of the compound to be tested was 20 mM, and the compound was diluted to a concentration of 20 μM in the 167 nM insulin medium.

Preparation of rapamycin solution: rapamycin was dissolved in DMSO to a concentration of 10 mM, and the rapamycin was diluted to a concentration of 20 μM in the 167 nM insulin medium.

⑤ The serum-free medium in each well was removed.

⑥ 2 mL of the complete medium containing DMSO was added to each well as a control carrier. The final concentration of DMSO was 0.2%.

⑦ 2 mL of 20 μM rapamycin solution and 2mL of 20 μM of the compound to be tested were separately added to the designated wells. The final concentration of DMSO was 0.2%.

⑧ The cells were incubated for 2 hours at 37°C and 5% $CO_2$.

Protein extraction and concentration determination:

① The cells were washed once with refrigerated PBS, and the PBS was discarded.

② 150 μL of the cell extraction buffer (RIPA, APPLYGEN, catalog number: C1053) was transferred into each well to lyse the cells, and then the resulting cell solution were incubated on ice for 30 minutes.

③ Centrifugation was performed at 14000 rpm (13000 × g) for 30 minutes at 4°C.

④ The supernatant was transferred into a new eppendorf tube, and the cell lysate was stored at -80°C before testing.

⑤ Protein concentration was determined by BCA method.

Preparation of buffer solution:

① 100×Protease inhibitor (Beyotime, catalog number: P1005): 1 mL of redistilled water was added to the protease inhibitor and stirred gently until the solid was completely dissolved.

② Lysis Buffer: 2 mL of 100× protease inhibitor and 2× phosphatase inhibitor Cocktails PhosSTOP (Beyotime, catalog number: P1082) were added to 100 mL of cell extract, and stirred gently until it was completely dissolved.

③ Electrophoresis Running Buffer:

10× MOPS buffer: 52.33 g of MOPS, 30.29 g of Tris base, 10 mL of 0.5 mol/L EDTA (pH 8.5), and 5 g of SDS were added and dissolved in 400 mL of redistilled water, stirred to dissolve, adjusted to have a pH of 7.5, and then the redistilled water was added to reach a volume of 500 mL;

1× MOPS: 100 mL of 10× MOPS was diluted to 1000 mL with redistilled water.

④ 1× Transfer Buffer: 100 mL of 10× transfer buffer (144g of glucine, 30.3g of trisbase, and distilled water were mixed to reach a volume of 1L) and 400 mL of methanol were dissolved in 1500 mL of redistilled water, and then the redistilled water was added to reach a volume of 2000 mL.

⑤ 10× PBS Buffer (0.1M): 5 bags of PBS powder (Solarbio, catalog number: P1010) were added to 800 mL of redistilled water, stirred to dissolve, adjusted to have a pH of 7.6, and then the redistilled water was added to reach a volume of 1000 mL.

⑥ 1× PBS Buffer: 100 mL of 10× PBS buffer was diluted to 1000 mL with redistilled water.

⑦ 10% Tween-20: 20 mL of Tween-20 was added to 180 mL of redistilled water, and stirred well.

⑧ 1× PBST Buffer: 100 mL of 10× PBST buffer and 10 mL of Tween-20 were mixed and diluted to 1000 mL with redistilled water.

⑨ Primary antibody incubation: the primary antibodies (Thermo Fisher, catalog numbers: B2H9L2 and PA5-85513) were diluted with 0.1% Tween-20 in the blocking solution (5% skimmed milk) at a ratio of 1:1000.

⑩ Secondary antibody incubation: IRDye 800CW Goat anti-Rabbit IgG (Abcam, catalog number: ab216773) was diluted with 0.1% Tween-20 in the blocking buffer at a ratio of 1:500.

Western Blot experiment:

① 12 μg of total protein was added to the sample well of SDS-PAGE. Electrophoresis was performed at a constant voltage of 120V until the blue marker reached the end of the gel.

② At 120 V, the protein on the gel was transferred to the PVDF membrane for 40 minutes by using the BIO-RAD Trans-Blot.

③ After transferring, the blocking buffer was used to perform blocking at room temperature for 2 hours.

④ The membrane was incubated with the corresponding primary antibody solution on a constant temperature shaker at 4°C overnight.

⑤ The membrane was rinsed with 1× PBST Buffer for 3×10 min, and then incubated with the secondary antibody solution at room temperature for 1 hour.

⑥ The membrane was washed with 1× PBST Buffer for 3×10 min, and scanned and developed with Odyssey Infrared Imaging System.

The RD cells were separately treated with 20 μM rapamycin solution and 20 μM the solution of compound of Formula I for 2 hours, and the Western blot results were shown in Figure 2.

The experimental results showed that under the same detection conditions, the cells treated with rapamycin and compound 9-(6-amino-pyridin-3-yl)-1-(3-trifluoromethyl-phenyl)-1H-benzo[h][1,6]naphthyridin-one had a significantly reduced phosphorylation expression levels of p70, indicating that both could inhibit mTORC1. On the contrary, under the same conditions, the compound could significantly down-regulate the phosphorylation level of Akt, while the positive control drug rapamycin could not inhibit the phosphorylation of Akt, indicating that compound 9-(6-amino-pyridin-3-yl)-1-(3-trifluoromethyl-phenyl)-1H-benzo[h][1,6]naphthyridin-one could effectively inhibit mTORC2 while inhibiting mTORC1, thereby exerting mTOR inhibition activity better, and then inhibiting virus replication and proliferation.

## Claims

1. A compound represented by Formula I, a pharmaceutically acceptable salt, a stereoisomer, a hydrate or a solvate thereof,

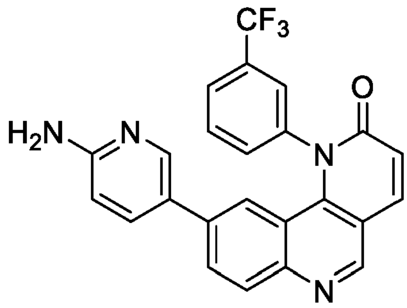

Formula I

for use in the treatment of an infectious disease caused by a virus, wherein the virus is an enterovirus, and wherein the enterovirus is an EV-D68, a Poliovirus I/II/III or a CB3 virus.

2. The compound represented by Formula I, a pharmaceutically acceptable salt, a stereoisomer, a hydrate or a solvate thereof for use according to claim 1, wherein the infectious disease is a hand-foot-and-mouth disease.

3. The compound, a pharmaceutically acceptable salt, a stereoisomer, a hydrate or a solvate thereof for use according to claim 1, wherein the pharmaceutically acceptable salt comprises salts formed with inorganic or organic acids and salts formed with inorganic or organic bases, such as: sodium salt, potassium salt, calcium salt, lithium salt, meglumine salt, hydrochloride, hydrobromide salt, hydriodide, nitrate, sulfate, hydrogen sulfate, phosphate, hydrogen phosphate, acetate, propionate, butyrate, oxalate, trimethylacetate, adipate, alginate, lactate, citrate, tartrate, succinate, maleate, fumarate, picrate, aspartate, gluconate, benzoate, methanesulfonate, ethanesulfonate, benzenesulfonate, p-toluenesulfonate and pamoate.

4. The compound, a pharmaceutically acceptable salt, a stereoisomer, a hydrate or a solvate thereof for use according to any one of claims 1 to 3, wherein the enterovirus is an EV-D68 or a CB3 virus.

5. A pharmaceutical composition comprising the compound of Formula I, a pharmaceutically acceptable salt, a stereoisomer, a hydrate or a solvate thereof according to claim 1,

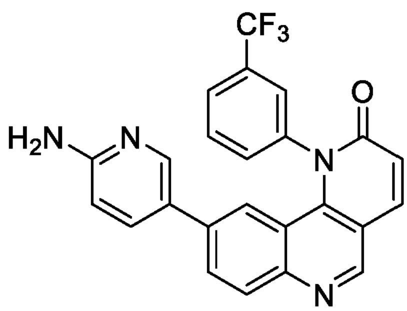

Formula I

for use in the treatment of an infectious disease caused by a virus, wherein the virus is an enterovirus, and wherein the enterovirus is an EV-D68, a Poliovirus I/II/III or a CB3 virus.

6. The pharmaceutical composition for use according to claim 5, wherein the pharmaceutical composition further comprises a pharmaceutically acceptable carrier or excipient.

7. The pharmaceutical composition for use according to claim 6, wherein the pharmaceutical composition is a solid

preparation, an injection, an external preparation, a spray, a liquid preparation, or a compound preparation.

8. The pharmaceutical composition for use according to any one of claims 5-7, wherein the infectious disease is a hand-foot-and-mouth disease.

9. The pharmaceutical composition for use according to any one of claims 5-7, wherein the pharmaceutically acceptable salt comprises salts formed with inorganic or organic acids and salts formed with inorganic or organic bases, such as: sodium salt, potassium salt, calcium salt, lithium salt, meglumine salt, hydrochloride, hydrobromide salt, hydriodide, nitrate, sulfate, hydrogen sulfate, phosphate, hydrogen phosphate, acetate, propionate, butyrate, oxalate, trimethylacetate, adipate, alginate, lactate, citrate, tartrate, succinate, maleate, fumarate, picrate, aspartate, gluconate, benzoate, methanesulfonate, ethanesulfonate, benzenesulfonate, p-toluenesulfonate and pamoate.

10. The pharmaceutical composition for use according to any one of claims 5 to 9, wherein the enterovirus is an EV-D68 or a CB3 virus.

## Patentansprüche

1. Verbindung, die durch Formel I dargestellt ist, ein pharmazeutisch akzeptables Salz, ein Stereoisomer, ein Hydrat oder ein Solvat davon,

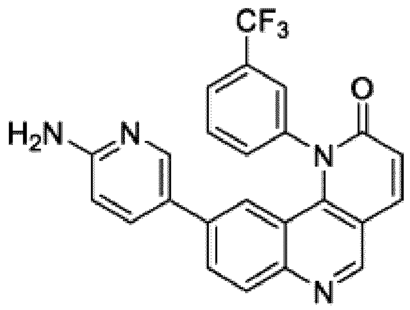

Formel I

zur Verwendung bei der Behandlung einer durch ein Virus verursachten Infektionskrankheit, wobei das Virus ein Enterovirus ist, und wobei das Enterovirus ein EV-D68, ein Poliovirus I/II/III oder ein CB3-Virus ist.

2. Verbindung, die durch Formel I dargestellt ist, ein pharmazeutisch akzeptables Salz, ein Stereoisomer, ein Hydrat oder ein Solvat davon zur Verwendung nach Anspruch 1, wobei die Infektionskrankheit eine Hand-Fuß- und Mund-Krankheit ist.

3. Verbindung, ein pharmazeutisch akzeptables Salz, ein Stereoisomer, ein Hydrat oder ein Solvat davon zur Verwendung nach Anspruch 1, wobei das pharmazeutisch akzeptable Salz Salze umfasst, die mit anorganischen oder organischen Säuren gebildet sind, und Salze, die mit anorganischen oder organischen Basen gebildet sind, wie: Natriumsalz, Kaliumsalz, Calciumsalz, Lithiumsalz, Megluminsalz, Hydrochlorid, Hydrobromidsalz, Hydriodid, Nitrat, Sulfat, Hydrogensulfat, Phosphat, Hydrogenphosphat, Acetat, Propionat, Butyrat, Oxalat, Trimethylacetat, Adipat, Alginat, Laktat, Citrat, Tartrat, Succinat, Maleat, Fumarat, Picrat, Aspartat, Gluconat, Benzoat, Methansulfonat, Ethansulfonat, Benzolsulfonat, p-Toluolsulfonat und Pamoat.

4. Verbindung, ein pharmazeutisch akzeptables Salz, ein Stereoisomer, ein Hydrat oder ein Solvat davon zur Verwendung nach einem der Ansprüche 1 bis 3, wobei der Enterovirus ein EV-D68 oder ein CB3-Virus ist.

5. Pharmazeutische Zusammensetzung, umfassend die Verbindung der Formel I, ein pharmazeutisch akzeptables Salz, ein Stereoisomer, ein Hydrat oder ein Solvat davon nach Anspruch 1,

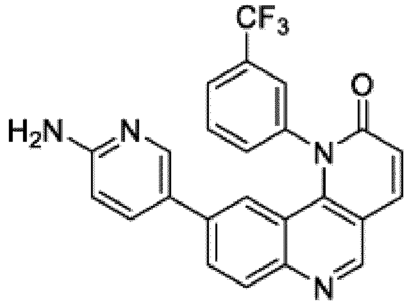

Formel I

zur Verwendung bei der Behandlung einer durch ein Virus verursachten Infektionskrankheit, wobei das Virus ein Enterovirus ist, und wobei das Enterovirus ein EV-D68, ein Poliovirus I/II/III oder ein CB3-Virus ist.

6. Pharmazeutische Zusammensetzung zur Verwendung nach Anspruch 5, wobei die pharmazeutische Zusammensetzung ferner einen pharmazeutisch akzeptablen Träger oder Hilfsstoff umfasst.

7. Pharmazeutische Zusammensetzung zur Verwendung nach Anspruch 6, wobei die pharmazeutische Zusammensetzung eine feste Zubereitung, eine Injektion, eine äußerliche Zubereitung, ein Spray, eine flüssige Zubereitung, oder eine zusammengesetzte Zubereitung ist.

8. Pharmazeutische Zusammensetzung zur Verwendung nach einem der Ansprüche 5-7, wobei die Infektionskrankheit eine Hand-Fuß- und Mund-Krankheit ist.

9. Pharmazeutische Zusammensetzung zur Verwendung nach einem der Ansprüche 5-7, wobei das pharmazeutisch akzeptable Salz Salze umfasst, die mit anorganischen oder organischen Säuren gebildet sind, und Salze, die mit anorganischen oder organischen Basen gebildet sind, wie: Natriumsalz, Kaliumsalz, Calciumsalz, Lithiumsalz, Megluminsalz, Hydrochlorid, Hydrobromidsalz, Hydriodid, Nitrat, Sulfat, Hydrogensulfat, Phosphat, Hydrogenphosphat, Acetat, Propionat, Butyrat, Oxalat, Trimethylacetat, Adipat, Alginat, Lactat, Citrat, Tartrat, Succinat, Maleat, Fumarat, Picrat, Aspartat, Gluconat, Benzoat, Methansulfonat, Ethansulfonat, Benzolsulfonat, p-Toluolsulfonat und Pamoat.

10. Pharmazeutische Zusammensetzung zur Verwendung nach einem der Ansprüche 5 bis 9, wobei der Enterovirus ein EV-D68 oder ein CB3-Virus ist.

**Revendications**

1. Composé représenté par la formule I, sel pharmaceutiquement acceptable, stéréoisomère, hydrate ou solvate de celui-ci,

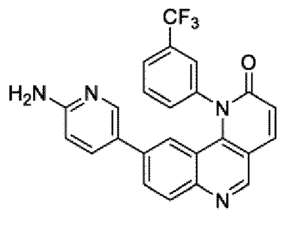

formule I

pour son utilisation dans le traitement d'une maladie infectieuse provoquée par un virus, dans lequel le virus est un entérovirus, et dans lequel l'entérovirus est un EV-D68, un poliovirus I/II/III ou un virus CB3.

2. Composé représenté par la formule I, sel pharmaceutiquement acceptable, stéréoisomère, hydrate ou solvate de celui-ci pour son utilisation selon la revendication 1, dans lequel la maladie infectieuse est une maladie pied-main-bouche.

3. Composé, sel pharmaceutiquement acceptable, stéréoisomère, hydrate ou solvate de celui-ci pour son utilisation selon la revendication 1, dans lequel le sel pharmaceutiquement acceptable comprend des sels formés avec des acides inorganiques ou organiques et des sels formés avec des bases inorganiques ou organiques, tels que : sel de sodium, sel de potassium, sel de calcium, sel de lithium, sel de méglumine, chlorhydrate, sel de bromhydrate, iodhydrate, nitrate, sulfate, sulfate d'hydrogène, phosphate, phosphate d'hydrogène, acétate, propionate, butyrate, oxalate, triméthylacétate, adipate, alginate, lactate, citrate, tartrate, succinate, maléate, fumarate, picrate, aspartate, gluconate, benzoate, méthanesulfonate, éthanesulfonate, benzènesulfonate, p-toluènesulfonate et pamoate.

4. Composé, sel pharmaceutiquement acceptable, stéréoisomère, hydrate ou solvate de celui-ci pour son utilisation selon l'une quelconque des revendications 1 à 3, dans lequel l'entérovirus est un EV-D68 ou un virus CB3.

5. Composition pharmaceutique comprenant le composé de formule I, un sel pharmaceutiquement acceptable, un stéréoisomère, un hydrate ou un solvate de celui-ci selon la revendication 1,

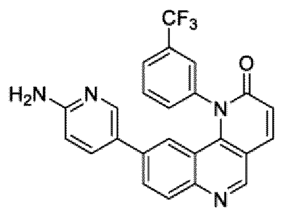

formule I

pour son utilisation dans le traitement d'une maladie infectieuse provoquée par un virus, dans lequel le virus est un entérovirus, et dans lequel l'entérovirus est un EV-D68, un poliovirus I/II/III ou un virus CB3.

6. Composition pharmaceutique pour son utilisation selon la revendication 5, dans laquelle la composition pharmaceutique comprend en outre un véhicule ou excipient pharmaceutiquement acceptable.

7. Composition pharmaceutique pour son utilisation selon la revendication 6, dans laquelle la composition pharmaceutique est une préparation solide, une injection, une préparation externe, une pulvérisation, une préparation liquide, ou une préparation composée.

8. Composition pharmaceutique pour son utilisation selon l'une quelconque des revendications 5 à 7, dans laquelle la maladie infectieuse est une maladie pied-main-bouche.

9. Composition pharmaceutique pour son utilisation selon l'une quelconque des revendications 5 à 7, dans laquelle le sel pharmaceutiquement acceptable comprend des sels formés avec des acides inorganiques ou organiques et des sels formés avec des bases inorganiques ou organiques, tels que : sel de sodium, sel de potassium, sel de calcium, sel de lithium, sel de méglumine, chlorhydrate, sel de bromhydrate, iodhydrate, nitrate, sulfate, sulfate d'hydrogène, phosphate, phosphate d'hydrogène, acétate, propionate, butyrate, oxalate, triméthylacétate, adipate, alginate, lactate, citrate, tartrate, succinate, maléate, fumarate, picrate, aspartate, gluconate, benzoate, méthanesulfonate, éthanesulfonate, benzènesulfonate, p-toluènesulfonate et pamoate.

10. Composition pharmaceutique pour son utilisation selon l'une quelconque des revendications 5 à 9, dans laquelle l'entérovirus est un EV-D68 ou un virus CB3.

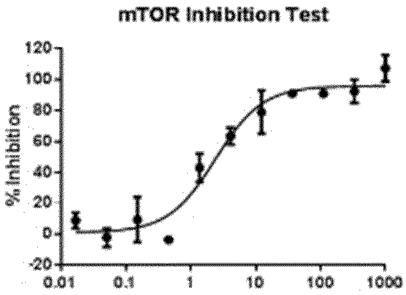


Figure 1

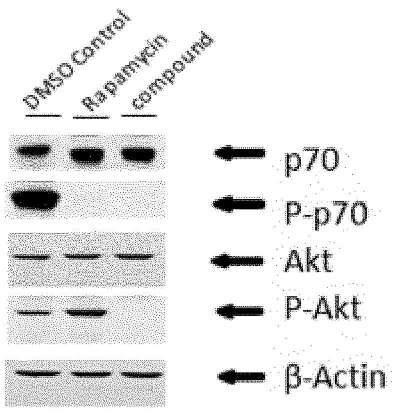


Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201910346258X


### Non-patent literature cited in the description


- **TIANLONG HAO et al.** *Eur. J. Med. Chem.,* 2019, 172-186